# EUROPEAN PATENT APPLICATION

(11) **EP 1 600 508 A1**
(43) Date of publication of application: **30.11.2005**
(21) Application number: 04713234.5
(22) Date of filing: 20.02.2004
(51) Int. Cl.: C12N 15/09, C12N 9/88, C12N 1/21, C12P 7/62

(54) **NOVEL VECTOR**

(30) Priority: 21.02.2003 JP 2003044136; 07.01.2004 JP 2004002334
(71) Applicant: KANEKA CORPORATION, Osaka-shi, Osaka 530-8288 (JP); RIKEN, Wako-shi, Saitama 351-0198 (JP)
(72) Inventor: OHNO, Emi, Settsu-shi, Osaka 566-0072 (JP); OKUBO, Yuji;, Takasago-shi, Hyogo 6760026; (JP); NAGAOKA, Tetsuya, Kobe-shi, Hyogo 6512124 (JP); DOI, Yoshiharu, Wako-shi, Saitama 3510198 (JP)
(74) Representative: HOFFMANN EITLE
(86) International application number: PCT/JP2004/002005
(87) International publication number: WO 2004/074476

(57) **Abstract**

The present invention provides a method for producing a polyester safely in an industrial production and/or efficiently in a low cost by a genetic engineering technique.

The present invention relates to a broad host range vector which is safer in an industrial production, a polyester synthase expression plasmid introduced with at least a polyester synthase gene to said vector, a transformant containing said plasmid and having polyester synthesis ability, and a method for producing a polyester using said plasmid or transformant.

SEQUENCE LISTING

<110>KANEKA CORPORATION
     RIKEN

<120>Novel vector

<130>B030079W001

<150>JP2003-044136
<151>2003-02-21

<150>JP2004-002334
<151>2004-01-07

<160>25

<210>1
<211>10316
<212>DNA
<213>Artificial Sequence

<220>
<223>plasmid pJRD215

<300>
<303>Gene
<304>51
<306>275-280
<307>1987

<400>1
aactgcacat tcgggatatt tctctatatt cgcgcttcat cagaaaactg aaggaacctc 60
cattgaatcg aactaatatt ttttttggtg aatcgcattc tgactggttg cctgtcagag 120
gcggagaatc tggtgatttt gtttttcgac gtggtgacgg gcatgccttc gcgaaaatcg 180

cacctgcttc ccgccgcggt gagctcgctg gagagcgtga ccgcctcatt tggctcaaag 240
gtcgaggtgt ggcttgcccc gaggtcatca actggcagga ggaacaggag ggtgcatgct 300
tggtgataac ggcaattccg ggagtaccgg cggctgatct gtctggagcg gatttgctca 360
aagcgtggcc gtcaatgggg cagcaacttg gcgctgttca cagcctatcg gttgatcaat 420
gtccgtttga gcgcaggctg tcgcgaatgt tcggacgcgc cgttgatgtg gtgtcccgca 480
atgccgtcaa tcccgacttc ttaccggacg aggacaagag tacgccgctg cacgatcttt 540
tggctcgtgt cgaacgagag ctaccggtgc ggctcgacca agagcgcacc gatatggttg 600
tttgccatgg tgatccctgc atgccgaact tcatggtgga ccctaaaact cttcaatgca 660
cgggtctgat cgaccttggg cggctcggaa cagcagatcg ctatgccgat ttggcactca 720
tgattgctaa cgccgaagag aactgggcag cgccagatga agcagagcgc gccttcgctg 780
tcctattcaa tgtattgggg atcgaagccc ccgaccgcga acgccttgcc ttctatctgc 840
gattggaccc tctgacttgg ggttgatgtt catgccgcct gtttttcctg ctcattggca 900
cgtttcgcaa cctgttctca ttgcggacac cttttccagc ctcgtttgga aagtttcatt 960
gccagacggg actcctgcaa tcgtcaaggg attgaaacct atagaagaca ttgctgatga 1020
actgcgcggg gccgactatc tggtatggcg caatgggagg ggagcagtcc ggttgctcgg 1080
tcgtgagaac aatctgatgt tgctcgaata tgccggggag cgaatgctct ctcacatcgt 1140
tgccgagcac ggcgactacc aggcgaccga aattgcagcg gaactaatgg cgaagctgta 1200
tgccgcatct gaggaacccc tgccttctgc ccttctcccg atccgggatc gctttgcagc 1260
tttgtttcag cgggcgcgcg atgatcaaaa cgcaggttgt caaactgact acgtccacgc 1320
ggcgattata gccgatcaaa tgatgagcaa tgcctcggaa ctgcgtgggc tacatggcga 1380
tctgcatcat gaaaacatca tgttctccag tcgcggctgg ctggtgatag atcccgtcgg 1440
tctggtcggt gaagtgggct ttggcgccgc caatatgttc tacgatccgg ctgacagaga 1500
cgacctttgt ctcgatccta gacgcattgc acagatggcg gacgcattct ctcgtgcgct 1560
ggacgtcgat ccgcgtcgcc tgctcgacca ggcgtacgct tatgggtgcc tttccgcagc 1620
ttggaacgcg gatggagaag aggagcaacg cgatctagct atcgcggccg cgatcaagca 1680
ggtgcgacag acgtcatact agatatcaag cgacttctcc tatcccctgg gaacacatca 1740
atctcaccgg agaatatcgc tggccaaagc cttagcgtag gattccgccc cttcccgcaa 1800
acgaccccaa acaggaaacg cagctgaaac gggaagctca acacccactg acgcatgggt 1860
tgttcaggca gtacttcatc aaccagcaag gcggcacttt cggccatccg ccgcgcccca 1920
cagctcgggc agaaaccgcg acgcttacag ctgaaagcga ccaggtgctc ggcgtggcaa 1980
gactcgcagc gaacccgtag aaagccatgc tccagccgcc cgcattggag aaattcttca 2040
aattcccgtt gcacatagcc cggcaattcc tttccctgct ctgccataag cgcagcgaat 2100
gccgggtaat actcgtcaac gatctgatag agaagggttt gctcgggtcg gtggctctgg 2160
taacgaccag tatcccgatc ccggctggcc gtcctggccg ccacatgagg catgttccgc 2220
gtccttgcaa tactgtgttt acatacagtc tatcgcttag cggaaagttc ttttaccctc 2280

agccgaaatg cctgccgttg ctagacattg ccagccagtg cccgtcactc ccgtactaac 2340
tgtcacgaac ccctgcaata actgtcacgc ccccctgcaa taactgtcac gaacccctgc 2400
aataactgtc acgcccccaa acctgcaaac ccagcagggg cgggggctgg cggggtgttg 2460
gaaaaatcca tccatgatta tctaagaata atccactagg cgcggttatc agcgcccttg 2520
tggggcgctg ctgcccttgc ccaatatgcc cggccagagg ccggatagct ggtctattcg 2580
ctgcgctagg ctacacaccg ccccaccgct gcgcggcagg gggaaaggcg ggcaaagccc 2640
gctaaacccc acaccaaacc ccgcagaaat acgctggagc gcttttagcc gctttagcgg 2700
cctttccccc tacccgaagg gtgggggcgc gtgtgcagcc ccgcagggcc tgtctcggtc 2760
gatcattcag cccggctcat ccttctggcg tggcggcaga ccgaacaagg cgcggtcgtg 2820
gtcgcgttca aggtacgcat ccattgccgc catgagccga tcctccggcc actcgctgct 2880
gttcaccttg gccaaaatca tggcccccac cagcaccttg cgccttgttt cgttcttgcg 2940
ctcttgctgc tgttcccttg cccgcacccg ctgaatttcg gcattgattc gcgctcgttg 3000
ttcttcgagc ttggccagcc gatccgccgc cttgttgctc cccttaacca tcttgacacc 3060
ccattgttaa tgtgctgtct cgtaggctat catggaggca cagcggcggc aatcccgacc 3120
ctactttgta ggggagggcg cacttaccgg tttctcttcg agaaactggc ctaacggcca 3180
cccttcgggc ggtgcgctct ccgagggcca ttgcatggag ccgaaaagca aaagcaacag 3240
cgaggcagca tggcgattta tcaccttacg gcgaaaaccg gcagcaggtc gggcggccaa 3300
tcggccaggg ccaaggccga ctacatccag cgcgaaggca agtatgcccg cgacatggat 3360
gaagtcttgc acgccgaatc cgggcacatg ccggagttcg tcgagcggcc cgccgactac 3420
tgggatgctg ccgacctgta tgaacgcgcc aatgggcggc tgttcaagga ggtcgaattt 3480
gccctgccgg tcgagctgac cctcgaccag cagaaggcgc tggcgtccga gttcgcccag 3540
cacctgaccg gtgccgagcg cctgccgtat acgctggcca tccatgccgg tggcggcgag 3600
aacccgcact gccacctgat gatctccgag cggatcaatg acggcatcga gcggcccgcc 3660
gctcagtggt tcaagcggta caacggcaag accccggaga agggcggggc acagaagacc 3720
gaagcgctca agcccaaggc atggcttgag cagacccgcg aggcatgggc cgaccatgcc 3780
aaccgggcat tagagcgggc tggccacgac gcccgcattg accacagaac acttgaggcg 3840
cagggcatcg agcgcctgcc cggtgttcac ctggggccga acgtggtgga gatggaaggc 3900
cggggcatcc gcaccgaccg ggcagacgtg gccctgaaca tcgacaccgc caacgcccag 3960
atcatcgact tacaggaata ccgggaggca atagaccatg aacgcaatcg acagagtgaa 4020
gaaatccaga ggcatcaacg agttagcgga gcagatcgaa ccgctggccc agagcatggc 4080
gacactggcc gacgaagccc ggcaggtcat gagccagacc cagcaggcca gcgaggcgca 4140
ggcggcggag tggctgaaag cccagcgcca gacaggggcg gcatgggtgg agctggccaa 4200
agagttgcgg gaggtagccg ccgaggtgag cagcgccgcg cagagcgccc ggagcgcgtc 4260
gcgggggtgg cactggaagc tatggctaac cgtgatgctg gcttccatga tgcctacggt 4320
ggtgctgctg atcgcatcgt tgctcttgct cgacctgacg ccactgacaa ccgaggacgg 4380

ctcgatctgg ctgcgcttgg tggcccgatg aagaacgaca ggactttgca ggccataggc 4440
cgacagctca aggccatggg ctgtgagcgc ttcgatatcg gcgtcaggga cgccaccacc 4500
ggccagatga tgaaccggga atggtcagcc gccgaagtgc tccagaacac gccatggctc 4560
aagcggatga atgcccaggg caatgacgtg tatatcaggc ccgccgagca ggagcggcat 4620
ggtctggtgc tggtggacga cctcagcgag tttgacctgg atgacatgaa agccgagggc 4680
cgggagcctg ccctggtagt ggaaaccagc ccgaagaact atcaggcatg ggtcaaggtg 4740
gccgacgccg caggcggtga acttcggggg cagattgccc ggacgctggc cagcgagtac 4800
gacgccgacc cggccagcgc cgacagccgc cactatggcc gcttggcggg cttcaccaac 4860
cgcaaggaca agcacaccac ccgcgccggt tatcagccgt gggtgctgct gcgtgaatcc 4920
aagggcaaga ccgccaccgc tggcccggcg ctggtgcagc aggctggcca gcagatcgag 4980
caggcccagc ggcagcagga gaaggcccgc aggctggcca gcctcgaact gcccgagcgg 5040
cagcttagcc gccaccggcg cacggcgctg gacgagtacc gcagcgagat ggccgggctg 5100
gtcaagcgct tcggtgatga cctcagcaag tgcgacttta tcgccgcgca gaagctggcc 5160
agccggggcc gcagtgccga ggaaatcggc aaggccatgg ccgaggccag cccagcgctg 5220
gcagagcgca agcccggcca cgaagcggat tacatcgagc gcaccgtcag caaggtcatg 5280
ggtctgccca gcgtccagct tgcgcgggcc gagctggcac gggcaccggc accccgccag 5340
cgaggcatgg acagggcggg gccagatttc agcatgtagt gcttgcgttg gtactcacgc 5400
ctgttatact atgagtactc acgcacagaa gggggtttta tggaatacga aaaaagcgct 5460
tcagggtcgg tctacctgat caaaagtgac aagggctatt ggttgcccgg tggctttggt 5520
tatacgtcaa acaaggccga ggctggccgc ttttcagtcg ctgatatggc cagccttaac 5580
cttgacggct gcaccttgtc cttgttccgc gaagacaagc ctttcggccc cggcaagttt 5640
ctcggtgact gatatgaaag accaaaagga caagcagacc ggcgacctgc tggccagccc 5700
tgacgctgta cgccaagcgc gatatgccga gcgcatgaag gccaaaggga tgcgtcagcg 5760
caagttctgg ctgaccgacg acgaatacga ggcgctgcgc gagtgcctgg aagaactcag 5820
agcggcgcag ggcgggggta gtgaccccgc cagcgcctaa ccaccaactg cctgcaaagg 5880
aggcaatcaa tggctaccca taagcctatc aatattctgg aggcgttcgc agcagcgccg 5940
ccaccgctgg actacgtttt gcccaacatg gtggccggta cggtcggggc gctggtgtcg 6000
cccggtggtg ccggtaaatc catgctggcc ctgcaactgg ccgcacagat tgcaggcggg 6060
ccggatctgc tggaggtggg cgaactgccc accggcccgg tgatctacct gcccgccgaa 6120
gacccgccca ccgccattca tcaccgcctg cacgcccttg gggcgcacct cagcgccgag 6180
gaacggcaag ccgtggctga cggcctgctg atccagccgc tgatcggcag cctgcccaac 6240
atcatggccc cggagtggtt cgacggcctc aagcgcgccg ccgagggccg ccgcctgatg 6300
gtgctggaca cgctgcgccg gttccacatc gaggaagaaa acgccagcgg ccccatggcc 6360
caggtcatcg gtcgcatgga ggccatcgcc gccgataccg ggtgctctat cgtgttcctg 6420
caccatgcca gcaagggcgc ggccatgatg ggcgcaggcg accagcagca ggccagccgg 6480

ggcagctcgg tactggtcga taacatccgc tggcagtcct acctgtcgag catgaccagc 6540
gccgaggccg aggaatgggg tgtggacgac gaccagcgcc ggttcttcgt ccgcttcggt 6600
gtgagcaagg ccaactatgg cgcaccgttc gctgatcggt ggttcaggcg gcatgacggc 6660
ggggtgctca agcccgccgt gctggagagg cagcgcaaga gcaagggggt gccccgtggt 6720
gaagcctaag aacaagcaca gcctcagcca cgtccggcac gacccggcgc actgtctggc 6780
ccccggcctg ttccgtgccc tcaagcgggg cgagcgcaag cgcagcaagc tggacgtgac 6840
gtatgactac ggcgacggca agcggatcga gttcagcggc ccggagccgc tgggcgctga 6900
tgatctgcgc atcctgcaag ggctggtggc catggctggg cctaatggcc tagtgcttgg 6960
cccggaaccc aagaccgaag gcggacggca gctccggctg ttcctggaac ccaagtggga 7020
ggccgtcacc gctgaatgcc atgtggtcaa aggtagctat cgggcgctgg caaaggaaat 7080
cggggcagag gtcgatagtg gtggggcgct caagcacata caggactgca tcgagcgcct 7140
ttggaaggta tccatcatcg cccagaatgg ccgcaagcgg caggggtttc ggctgctgtc 7200
ggagtacgcc agcgacgagg cggacgggcg cctgtacgtg gccctgaacc ccttgatcgc 7260
gcaggccgtc atgggtggcg gccagcatgt gcgcatcagc atggacgagg tgcgggcgct 7320
ggacagcgaa accgcccgcc tgctgcacca gcggctgtgt ggctggatcg accccggcaa 7380
aaccggcaag gcttccatag ataccttgtg cggctatgtc tggccgtcag aggccagtgg 7440
ttcgaccatg cgcaagcgcc gccagcgggt gcgcgaggcg ttgccggagc tggtcgcgct 7500
gggctggacg gtaaccgagt tcgcggcggg caagtacgac atcacccggc ccaaggcggc 7560
aggctgaccc cccccactct attgtaaaca agacattttt atcttttata ttcaatggct 7620
tattttcctg ctaattggta ataccatgaa aaataccatg ctcagaaaag gcttaacaat 7680
attttgaaaa attgcctact gagcgctgcc gcacagctcc ataggccgct ttcctggctt 7740
tgcttccaga tgtatgctct tctgctcctg cagttcgggg gcatggatgc gcggatagcc 7800
gctgctggtt tcctggatgc cgacggattt gcactgccgg tagaactccg cgaggtcgtc 7860
cagcctcagg cagcagctga accaactcgc gaggggatcg agcccggggt gggcgaagaa 7920
ctccagcatg agatccccgc gctggaggat catccagccg gcgtcccgga aaacgattcc 7980
gaagcccaac ctttcataga aggcggcggt ggaatcgaaa tctcgtgatg gcaggttggg 8040
cgtcgcttgg tcggtcattt cgcctcgagg cgaaccccag agtcccgctc agaagaactc 8100
gtcaagaagg cgatagaagg cgatgcgctg cgaatcggga gcggcgatac cgtaaagcac 8160
gaggaagcgg tcagcccatt cgccgccaag ctcttcagca atatcacggg tagccaacgc 8220
tatgtcctga tagcggtccg ccacacccag ccggccacag tcgatgaatc cagaaaagcg 8280
gccattttcc accatgatat tcggcaagca ggcatcgcca tgggtcacga cgagatcctc 8340
gccgtcgggc atgcgcgcct tgagcctggc gaacagttcg gctggcgcga gcccctgatg 8400
ctcttcgtcc agatcatcct gatcgacaag accggcttcc atccgagtac gtgctcgctc 8460
gatgcgatgt ttcgcttggt ggtcgaatgg gcaggtagcc ggatcaagcg tatgcagccg 8520
ccgcattgca tcagccatga tggatacttt ctcggcagga gcaaggtgag atgacaggag 8580

atcctgcccc ggcacttcgc ccaatagcag ccagtccctt cccgcttcag tgacaacgtc 8640
gagcacagct gcgcaaggaa cgcccgtcgt ggccagccac gatagccgcg ctgcctcgtc 8700
ctgcagttca ttcagggcac cggacaggtc ggtcttgaca aaaagaaccg ggcgcccctg 8760
cgctgacagc cggaacacgg cggcatcaga gcagccgatt gtctgttgtg cccagtcata 8820
gccgaatagc ctctccaccc aagcggccgg agaacctgcg tgcaatccat cttgttcaat 8880
catgcgaaac gatcctcatc ctgtctcttg atcagatctt gatcccctgc gccatcagat 8940
ccttggcggc aagaaagcca tccagtttac tttgcagggc ttcccaacct taccagaggg 9000
cgccccagct ggcaattccg gttcgcttgc tgtccataaa accgcccagt ctagctatcg 9060
ccatgtaagc ccactgcaag ctacctgctt tctctttgcg cttgcgtttt cccttgtcca 9120
gatagcccag tagctgacat tcatccgggg tcagcaccgt ttctgcggac tggctttcta 9180
cgtgttccgc ttcctttagc agcccttgcg ccctgagtgc ttgcggcagc gtgaagctag 9240
cttggctgca gggggggggg ggggacttcc attgttcatt ccacggacaa aaacagagaa 9300
aggaaacgac agaggccaaa aagcctcgct ttcagcacct gtcgtttcct ttcttttcag 9360
agggtatttt aaataaaaac attaagttat gacgaagaag aacggaaacg ccttaaaccg 9420
gaaaattttc ataaatagcg aaaacccgcg aggtcgccgc cccgtaacct gtcggatcac 9480
cggaaaggac ccgtaaagtg ataatgatta tcatctacat atcacaacgt gcgtggaggc 9540
catcaaacca cgtcaaataa tcaattatga cgcaggtatc gtattaattg atctgcatca 9600
acttaacgta aaaacaactt cagacaatac aaatcagcga cactgaatac ggggcaacct 9660
catgtccccc cccccccctg caggtcgacg gatccccggg taccataagt agaagcagca 9720
acccaagtag ctttaccagc atccggttca accagcatag taagaatctt actggacatc 9780
ggcagttctt cgaacagtgc gccaactacc agctctttct gcagttcatt cagggcaccg 9840
gagaacctgc gtgcaatcca tcttgttcaa tcatgcgaaa cgatcctcat cctgtctctt 9900
gatccatgga ttacgcgtta acccgggccc gcggatgcat atgatcttaa ggcctaggtc 9960
tagagtcttt gttttgacgc cattagcgta cgtaacaatc ctcgttaaag gacaaggacc 10020
tgagcggaag tgtatcgtac agtagacgga gtatactagt atagtctata gtccgtggaa 10080
ttattatatt tatctccgac gatattctca tcagtgaaat ccaggggaat tctcatgttt 10140
gacagcttat catcgataag ctttaatgcg gtagtttatc acagttaaat tgctaacgca 10200
gtcaggcacc gtgtatgaaa tctaacaatg cgctcatcgt catcctcggc accgtcaccc 10260
tggatgctgt aggcataggc ttggttatgc cggtactgcc gggcctcttg cgggat 10316

<210>2
<211>45
<212>DNA
<213>Artificial Sequence

<220>
<223>primer

<400>2
ctagcatgcg gatccacggt tatccgaatt cgcgtgctta gcggg 45

<210>3
<211>45
<212>DNA
<213>Artificial Sequence

<220>
<223>primer

<400>3
aattcccgct aagcacgcga attcggataa ccgtggatcc gcatg 45

<210>4
<211>24
<212>DNA
<213>Artificial Sequence

<220>
<223>primer

<400>4
aaatacgctg gagcgctttt agcc 24

<210>5
<211>24
<212>DNA

<213>Artificial Sequence

<220>
<223>primer

<400>5
tgatatacac gtcattgccc tggg                                          24


<210>6
<211>35
<212>DNA
<213>Artificial Sequence

<220>
<223>primer

<400>6
tggccgttag gctacaaagt agggtcggga ttgcc                              35


<210>7
<211>33
<212>DNA
<213>Artificial Sequence

<220>
<223>primer

<400>7
ctactttgta gcctaacggc cacccttcgg gcg                               33


<210>8
<211>34

<212>DNA
<213>Artificial Sequence

<220>
<223>primer

<400>8
gaaaccggta agtctacaaa gtagggtcgg gatt                                    34



<210>9
<211>41
<212>DNA
<213>Artificial Sequence

<220>
<223>primer

<400>9
ccctactttg tagacttacc ggtttctctt cgagaaactg g                            41



<210>10
<211>40
<212>DNA
<213>Artificial Sequence

<220>
<223>primer

<400>10
aattaatcca tagcatgggg ctcgatctgg ctgcgcttgg                              40



<210>11

<211>33
<212>DNA
<213>Artificial Sequence

<220>
<223>primer

<400>11
aattctcgag tgtttcgttc ttgcgctctt gct                                    33


<210>12
<211>33
<212>DNA
<213>Artificial Sequence

<220>
<223>primer

<400>12
aattctgcag gtcgttcttc atcgggccac caa                                    33


<210>13
<211>26
<212>DNA
<213>Artificial Sequence

<220>
<223>primer

<400>13
aagcgctcaa gccaaaggca tggctt                                            26

<210>14
<211>26
<212>DNA
<213>Artificial Sequence

<220>
<223>primer

<400>14
aagccatgcc tttggcttga gcgctt 26

<210>15
<211>25
<212>DNA
<213>Artificial Sequence

<220>
<223>primer

<400>15
gagggccata gcatggagcc gaaaa 25

<210>16
<211>30
<212>DNA
<213>Artificial Sequence

<220>
<223>primer

<400>16
atttaagcca tgctatgggc ttgagcgctt 30

<210>17
<211>36
<212>DNA
<213>Artificial Sequence

<220>
<223>primer

<400>17
aaattaacca tgctatggtc tattgcctcc cggtat 36

<210>18
<211>26
<212>DNA
<213>Artificial Sequence

<220>
<223>primer

<400>18
ttcgcgctgg atgaagtcgg ccttgg 26

<210>19
<211>26
<212>DNA
<213>Artificial Sequence

<220>
<223>primer

<400>19
ccaaggccga cttcatccag cgcgaa 26

<210>20
<211>25
<212>DNA
<213>Artificial Sequence

<220>
<223>primer

<400>20
taacgaccag tatcccgatc ccggc 25

<210>21
<211>36
<212>DNA
<213>Artificial Sequence

<220>
<223>primer

<400>21
ccatgcaatg gccatgatag cctacgagac agcaca 36

<210>22
<211>40
<212>DNA
<213>Artificial Sequence

<220>
<223>primer

<400>22

attccatgca atgggggatt gccgccgctg tgcctccatg 40

<210>23
<211>100
<212>DNA
<213>Artificial Sequence

<220>
<223>primer

<400>23
tccatgcaat ggccctcgga gagcgcaccg cccgaagggt ggccgttagg ccagtttctc 60
gaagagaaac cggtaagtgc ctacaaagta gggtcgggat 100

<210>24
<211>26
<212>DNA
<213>Artificial Sequence

<220>
<223>primer

<400>24
accctggagt ccggcggcca gaacct 26

<210>25
<211>26
<212>DNA
<213>Artificial Sequence

<220>
<223>primer

<400>25
aggttctggc cgccggactc cagggt 26

## Description

### TECHNICAL FIELD

The present invention relates to a novel vector produced by modifying a broad host range vector "pJRD215 vector" to improve them, and a method for producing a polyester using the same.

### BACKGROUND ART

Up to present, a large number of microorganisms have been known to store polyesters as an energy source substance within cells. A typical example of the polyester is poly-3-hydroxybutyric acid (hereinafter referred to briefly as "P(3HB)"), which is a homopolymer of 3-hydroxybutyric acid (hereinafter referred to briefly as "3HB"). It was first discovered in Bacillus megaterium in 1925. P(3HB) is a thermoplastic polymer and is biodegradable in the natural environment and, thus, has recently attracted attention as an eco-friendly plastic. However, since P(3HB) is high in crystallinity, it is hard and brittle by nature, so that the range of practical application thereof is limited. Therefore, studies have been undertaken to modify the P(3HB) for improving these properties.

In the course of the study, a technology of producing a copolymer composed of 3-hydroxybutyric acid (3HB) and 3-hydroxyvaleric acid (3HV) (hereinafter such copolymers are referred to briefly as "P(3HB-co-3HV)") has been disclosed (see, for example, Japanese Kokai Publication Sho-57-150393 and Japanese Kokai Publication Sho-59-220192). This P(3HB-co-3HV) is rich in flexibility as compared with P(3HB), hence was considered to have a wide application range. Actually, however, P(3HB-co-3HV) shows only slight changes in physical properties even when the mole fraction of 3HV is increased. In particular, the flexibility, which is required for its use in films and the like, is not improved. Thus, it has been used only in the field of rigid shaped articles such as shampoo bottles and disposable razor grips.

In recent years, studies have been made concerning two component copolyesters composed of 3HB and 3-hydroxyhexanoic acid (hereinafter referred to briefly as "3HH") (hereinafter such copolyesters are referred to briefly as "P(3HB-co-3HH)"), and a method for producing the same (see, for example, Japanese Kokai Publication Hei-05-93049 and Japanese Kokai Publication Hei-07-265065). The methods described in these publications directed to technologies of producing P(3HB-co-3HH) comprises using Aeromonas caviae isolated from soil to carry out fermentative production thereof from fatty acids such as oleic acid, or oils and fats such as olive oil. Studies concerning properties of P(3HB-co-3HH) have also been made (see, for example, Y. Doi, S. Kitamura, H. Abe, Macromolecules 28, 4822-4823 (1995)). According to this report, Aeromonas caviae is cultured using a fatty acid containing not less than 12 carbon atoms as an only carbon source, and P(3HB-co-3HH) with a 3HH content of 11 to 19 mole% is fermentatively produced. It has been revealed that the properties of such P(3HB-co-3HH) change from hard and brittle gradually to soft and flexible, to an extent exceeding the flexibility of P(3HB-co-3HV), with the increase in mole fraction of 3HH. However, the above method of production is low in productivity, namely the yield of cells is 4 g/L and the polymer content is 30%. Therefore, methods capable of attaining higher productivity for practical use have been searched for.

Since said P(3HB-co-3HH) can have a wide range of properties from hard polymers to soft polymers by controlling the 3HH molar fraction, such copolymers will find a broad range of applications, from products required to be hard, such as a chassis for a TV-set, to products required to be flexible, such as a thread or a film. However, productivity of the polymer remains still low in these production methods, thus it would have to be said that these methods are inadequate as methods for aiming practical use of this polymer.

A poly hydroxyalkanoic acid (PHA) synthase gene was cloned from Aeromonas caviae producing P(3 HB-co-3HH) (see, for example, Japanese Kokai Publication Hei-10-108682 and T. Fukui, Y. Doi, J. Bacteriol, 179, 15, 4821-4830 (1997)). As a result of producing P(3HB-co-3HH) using a transformant obtained by introducing said gene to Ralstonia eutropha (old name: Alcaligenes eutrophus), cell productivity was 4 g/L and a polymer content was 30%. Moreover, as a result of culturing this transformant using a vegetable oil or fat as a carbon source, a cell content of 4 g/L and a polymer content of 80% were achieved (see, for example, T. Hukui et al. Appl. Microbiol. Biotechnol. 49, 333 (1998)). PHA synthase expression plasmids used in this investigation were pJRDEE32, pJRDEE32d13, etc. which had been obtained by introducing a polyester synthase gene into pJRD215 (ATCC 37533) (see, for example, Japanese Kokai Publication Hei-10-108682). Thereafter, many kinds of modified PHA synthases have been constructed based on the above synthase. Among them, it is reported that a modified enzyme, wherein serine is substituted for 149th amino acid asparagine, and a modified enzyme, wherein glycine is substituted for 171st amino acid aspartic acid, are improved in their PHA synthase activity in Escherichia coli and the composition of 3HH produced (T. Kichise et al., Appl. Environ. Microbiol. 68, 2411-2419 (2002)).

PJRD215 is a derivative of RSF1010, a broad host range vector, and has an ability of conjugal mobilization (see, for example, J. Davison, M. Heusterspreute et al., Gene, 51, 275-280 (1987)).

Conjugal mobilization is a phenomenon which occurs when bacteria having different characteristics are mixed and cultured, and means that part of genes of a certain bacterium (donor bacterium) transfers to another bacterium (recipient bacterium). Strength of an ability of conjugal mobilization is determined depending on a gene involving with conjugal mobilization on a chromosome of a donor or on a plasmid which the donor has. Genes involving with conjugal mobilization include a self-mobilized gene "tra", a conjugate-mobilized gene "mob", and an "oriT sequence". A protein encoded by the tra involves with an interaction between a donor bacterium and a recipient bacterium. And a protein encoded by the mob functions as inserting a nick to the oriT sequence, and stably transmitting a single-strand DNA. The oriT sequence is composed of a nick site and a recognition sequence for the nick to be inserted. Conjugal mobilization takes place only when these three species of genes coexist.

Researches on RSF1010, which was used for construction of pJRD215, have been intensively carried out heretofore. It is already known that the copy number of plasmids replicated in Esherichia coli is increased by a partial deletion of genes involving with an ability of conjugal mobilization, such as a partial deletion of a mobB gene, partial deletions of both mobA and mobB genes, or a partial deletion of an oriT sequence, and then most of an ability of conjugal mobilization is lost (see, for example, J. Frey, M. M. Bagdasarian, M. Bagdasarian, Gene, 113, 101-106 (1992)). Furthermore, in researches using a plasmid R1162 showing high homology with an oriT region and a mobA gene region of RSF1010, it was reported that functions of a mob protein were inactivated by deleting amino acids in mobA genes of the 180th or later, or by substituting the 25th amino acid residue tyrosine of a mobA gene with phenylalanine (see, for example, E. C. Becker, R. J. Meyer, J. Biol. Chem, 277, 14575-14580 (2002)).

However, the mobA gene of RSF1010 partially overlaps with a repB gene involving with replication (see, for example, P. Scholz, V. Haring et al., Gene, 75, 271-288 (1989)). Therefore, it was reported that, in Pseudomonas putida, a vector had not been replicated when mobA and mobB genes had been partially deleted (see, for example, M. M. Bagdasarian, P. Scholz et al., Banbury Report, 24, 209-223 (1986)). Accordingly, it has been unclear if such a modified vector might be replicated in Ralstonia eutropha.

pJRD215 has a mobB gene region and an oriT sequence among the genes involving with conjugal mobilization. Heretofore, if, by any possibility, a transformant harboring pJRD215 is leaked in producing a polyester on an industrial scale, and is contacted with a microorganism harboring a tra gene-containing plasmid such as RP4, conjugal mobilization may possibly occur. Therefore, there was a security problem such as containment of a recombinant organism.

Additionally, as a transformation method of Ralstonia eutropha PHB-4 strain (DSM541) (polyester synthesis ability-defected strain), the above-mentioned conjugal mobilization methods have mainly been used. Therefore, when an ability of conjugal mobilization is lost, it becomes necessary to carry out transformation using other methods than the conjugal mobilization method, i.e., an electroporation method, a calcium method, etc. However, when introducing the pJRD215 vector into the host Ralstonia eutropha PHB-4 strain, it was experientially known that transformation efficiency would be remarkably decreased because of its plasmid size.

Thus, when an ability of conjugal mobilization is lost, another problem, i.e. an improvement of a polyester synthase expression plasmid, arises on transformation of a host. Therefore, when introducing said vector into the host Ralstonia eutropha PHB-4 strain, using an electroporation method, etc., reduction of plasmid size has been desired for improvement of transformation efficiency.

### SUMMARY OF THE INVENTION

The present inventors have carried out an intensive investigation to solve the above-mentioned problems. As a result, they found that an ability of conjugal mobilization of a vector is remarkably reduced by partially deleting or modifying mob genes and/or by partially or totally deleting an oriT sequence from the broad host range vector pJRD215, which have been used conventionally, and that a vector capable of being used more safely in an industrial production may be thereby obtained.

Moreover, they also found that a small size vector having improved transformation efficiency may be obtained by deleting unnecessary gene sequence parts, i.e., a streptomycin resistance gene and a cos sequence, from the broad host range vector pJRD215 for improvement of operability.

Furthermore, they succeeded in producing a polyester using a transformant derived from those improved vectors. Namely, it was a method of producing a polyester which comprises inserting an enzyme expression unit (EE32, EE32d13, N149S, D171G, F353T, etc.), which synthesizes P(3HB-co-3HH) copolymer derived from Aeromonas caviae, into said improved vector to obtain an expression plasmid, then introducing the expression plasmid into Ralstonia eutropha, and employing thus-obtained said transformant for production of a polymer. Based on these findings, the present invention has now completed.

That is, the present invention relates to a broad host range vector which can be used more safely in an industrial production, a polyester synthase expression plasmid obtainable by introducing at least a polyester synthase gene into said vector, a transformant containing said plasmid and having polyester synthesis ability, and a method for producing a polyester using said plasmid.

That is, the present invention relates to;
a polyester synthase expression plasmid
which has partly or totally lost ability of conjugal mobilization;
a polyester synthase expression plasmid
which comprises a deletion of a streptomycin resistance gene region;
a polyester synthase expression plasmid
which has partly or totally lost ability of conjugal mobilization, and comprises a deletion of a streptomycin resistance gene;
a transformant
which is transformed with the above-mentioned polyester synthase expression plasmid; and
a method for producing a polyester
which comprises using the above expression plasmid or the above transformant,
said polyester being a copolyester P(3 HB-co-3HH) composed of 3-hydroxybutyric acid and 3-hydroxyhexanoic acid represented by the following formula (1); 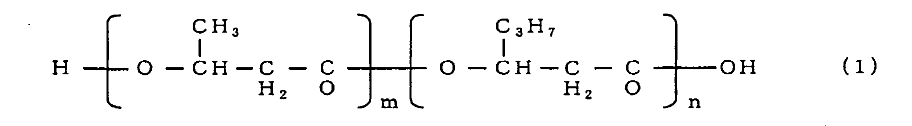 in the formula, m and n each represents an integer of 1 or more.

### DETAILED DESCRIPTION OF THE INVENTION

Hereinafter, the present invention is described in detail.

The polyester synthase expression plasmid of the present invention is;
a polyester synthase expression plasmid
which has partly or totally lost ability of conjugal mobilization;
a polyester synthase expression plasmid
which comprises a deletion of a streptomycin resistance gene region; and
a polyester synthase expression plasmid
which has partly or totally lost ability of conjugal mobilization, and comprises a deletion of a streptomycin resistance gene.

### Production of a vector

Firstly, production of a vector is explained.

Overall gene manipulation may be carried out as described in Molecular Cloning (Cold Spring Harbor Laboratory Press, 1989). Enzymes and a cloning host to be used for gene operation may be purchased from market suppliers, and used according to their instruction. The enzymes are not particularly restricted as long as those are usable for gene operation. The cloning host is not also particularly restricted, but there may be mentioned, for example, Escherichia coli, etc.

The vector to be used in the present invention is not particularly restricted as long as it is a broad host range vector having a conjugal mobilization gene, but preferably pJRD215 disclosed in J. Davison, M. Heusterspreute et al., Gene, 51, 275-280 (1987) may be used. A base sequence of pJRD215 is shown under the SEQ ID NO:1 of the sequence listing.

An ability of conjugal mobilization of the vector may be lowered or lost by carrying out any of a total deletion, a partial deletion, a base insertion, a base substitution, etc. of a gene region involving with conjugal mobilization property.

For example, if it is pJRD215, any of a total deletion, a partial deletion, a base insertion, a base substitution, etc. is carried out for mobA, mobB, mobC genes and an oriT sequence. Although any functions of a gene among the mobA, the mobB, the mobC and the oriT sequence may be lost, it is particularly preferable to carry out the above-mentioned operation for the mobA gene, which is indispensable for mobility, or the oriT sequence, which has a nick site. Furthermore, the gene to be lowered or lost its function may be one genes, but more preferably two or more genes.

In addition, since pJRD215 is a derivative of RSF1010, a definition of RSF1010 described in P. Scholz, V. Haring et al., Gene, 75, 271-288 (1989) may be cited as it is. That is, the oriT sequence of pJRD215 may be defined as the sequence from 3081 to 3169 of the SEQ ID NO:1 of the sequence listing, and the nick site may be defined as the sequence between 3138 and 3139. The mobA gene may be defined as the sequence from 3250 to 4407 of the SEQ ID NO:1 of the sequence listing, and the mobB gene may be defined as the sequence from 3998 to 4411 of the SEQ ID NO:1 of the sequence listing.

As a polyester synthase expression plasmid in which partial or total loss of an ability of conjugal mobilization is caused by a deletion or mutation of its mob gene region, there may be mentioned, for example, a polyester synthase expression plasmid in which the sequence from 3215 to 4075 of pJRD215 (SEQ ID NO:1) is deleted; a polyester synthase expression plasmid in which the sequence from 3737 to 4378 of pJRD215 is deleted; a polyester synthase expression plasmid in which the sequence from 4000 to 4378 of pJRD215 is deleted; a polyester synthase expression plasmid in which thymine is substituted for 3323 adenine of pJRD215; etc.

Moreover, as a polyester synthase expression plasmid in which partial or total loss of an ability of conjugal mobilization is caused by a deletion of its oriT region, there may be mentioned, for example, a polyester synthase expression plasmid in which the sequence from 3132 to 3145 of pJRD215 (SEQ ID NO:1) is deleted; a polyester synthase expression plasmid in which the sequence from 3132 to 3169 of pJRD215 is deleted; etc.

Furthermore, as a polyester synthase expression plasmid in which partial or total loss of an ability of conjugal mobilization are caused by deletions of its mob gene region and oriT region, there may be mentioned, for example,
a polyester synthase expression plasmid
wherein the deletion of a mob gene region is a deletion of the sequence from 3215 to 4075 of pJRD215 (SEQ ID NO:1), and the deletion of an oriT region is a deletion of the sequence from 3132 to 3145 of pJRD215 (SEQ ID NO:1);
a polyester synthase expression plasmid
wherein the deletion of a mob gene region is a deletion of the sequence from 3215 to 4075 of pJRD215, and the deletion of an oriT region is a deletion of the sequence from 3132 to 3169 of pJRD215;
a polyester synthase expression plasmid
wherein the deletion of a mob gene region is a deletion of the sequence from 3215 to 4075 of pJRD215, and the deletion of an oriT region is a deletion of the sequence from 3132 to 3178 of pJRD215;
a polyester synthase expression plasmid
wherein the deletion of a mob gene region is a deletion of the sequence from 3215 to 4075 of pJRD215, and the deletion of an oriT region is a deletion of the sequence from 3132 to 3214 of pJRD215;
a polyester synthase expression plasmid
wherein the deletion of a mob gene region is a deletion of the sequence from 3215 to 4075 of pJRD215, and the deletion of an oriT region is a deletion of the sequence from 3095 to 3214 of pJRD215; etc.

The deletion of a mob gene region may be carried out by deleting a restriction fragment containing a mob gene, deleting using PCR, or the like. The mutation of a mob gene region may be carried out by a site-directed mutagenesis, etc. And the deletion of an oriT region may be carried out by deleting a restriction fragment containing an oriT part, deleting using PCR, etc., as in the deletion of a mob gene region mentioned above.

Reduction of the size of a plasmid for improvement of transformation efficiency can be carried out by a deletion of parts which are unnecessary for expression of a polyester synthase gene and for replication of the plasmid. For example, if there are two or more antibiotic resistance genes in a vector to be used, any one of them may be deleted.

For example, in the case of pJRD215, it is possible to delete a kanamycin resistance gene or a streptomycin resistance gene, but it is preferable to delete the streptomycin resistance gene.

Moreover, in the case of pJRD215, it is possible to delete also a multicloning site, cos region, etc., which are not involved in plasmid replication for downsizing the plasmid. Although a multicloning site and a λ phage-derived cos region are not defined clearly, it may be understood that the vicinity of the sequence from 9680 to 10130 of the SEQ ID NO:1 in the sequence listing is the multicloning site, and the vicinity of the sequence from 9260 to 9660 is the cos region from J. Davison, M. Heusterspreute et al. Gene, 51, 275-280 (1987).

As the polyester synthase expression plasmid which has a deleted streptomycin resistance gene region, there may be mentioned, for example, a polyester synthase expression plasmid in which the sequence from 206 to 1690 of pJRD215 (SEQ ID NO:1) is deleted, etc.

As the polyester synthase expression plasmid which has a deleted cos region, there may be mentioned, for example, a polyester synthase expression plasmid in which the sequence from 9237 to 10127 of pJRD215 (SEQ ID NO:1) is deleted; a polyester synthase expression plasmid in which the sequence from 8915 to 10055 of pJRD215 is deleted, etc.

The deletions of a streptomycin resistance gene region, a cos region, and a multicloning site may be carried out in the same manner as the deletion of a mob gene region, mentioned above.

Additionally, a polyester synthase expression plasmid, which has partly or totally lost ability of conjugal mobilization and comprises a deletion of a streptomycin resistance gene, may be preferably used. Said polyester synthase expression plasmid may be obtained by a manner to lose part or total of an ability of conjugal mobilization in combination with the deletion of a streptomycin resistance gene, which are mentioned above.

It is also possible to further combine the deletion of a cos region and the deletion of a multicloning site. For example, there may be mentioned, a polyester synthase expression plasmid in which part or total of an ability of conjugal mobilization is lost, and a cos region and/or a multicloning site are deleted; a polyester synthase expression plasmid in which a streptomycin resistance gene is deleted, and a cos region and/or a multicloning site are deleted; a polyester synthase expression plasmid in which part or total of an ability of conjugal mobilization is lost, a streptomycin resistance gene is deleted, and a cos region and/or a multicloning site are deleted; etc.

Next, production of a polyester synthase expression plasmid is explained.

The polyester synthase expression plasmid of the present invention may be produced by inserting a polyester synthase gene into the vector produced as mentioned above.

As the polyester synthase gene, those of having an expression unit functioning in host bacteria, such as a promoter, a terminator, etc. together with a structural gene may be used.

The polyester synthase gene is preferably derived from Aeromonas caviae, and in particular, for example, gene fragments derived from Aeromonas caviae, such as EE32 and EE32d13, disclosed in Japanese Kokai Publication Hei-10-108682, and the like gene may be used. Furthermore, preferably used are an Aeromonas caviae-derived polyester synthase gene (N149S-modified gene), wherein serine is substituted for 149th amino acid asparagine, and an Aeromonas caviae-derived polyester synthase gene (D171G-modified gene), wherein glycine is substituted for 171st amino acid aspartic acid, those two are described in T. Kichise et al., Appl. Environ. Microbial. 68, 2411-2419 (2002), and an Aeromonas caviae-derived polyester synthase gene (F353T-modified gene), wherein threonine is substituted for 353rd amino acid phenylalanine and which has been designated by a program (e.g. Shrike, Japanese Kokai Publication 2001-184831) capable of determining an useful amino acid modification based on the computer-aided enzyme conformation or a predicted conformation, and the like program, and an Aeromonas caviae-derived polyester synthase gene (a modified gene, comprising a combination of the above modifications), comprising two or more of the above amino acid substitutions, and the like gene. Additionally, the above polyester synthase expression plasmid may comprise one or plural of said expression units.

### Construction of a transformant

Next, construction of a transformant is explained.

The transformant of the present invention is transformed by the above polyester synthase expression plasmid. That is, the transformant of the present invention may be obtained by transforming the polyester synthase expression plasmid obtained as above into a host compatible with said plasmid.

The host is not particularly restricted, but microorganisms isolated from nature or those deposited to depository institutions of strains (for example, IFO, ATCC, etc.), etc. may be used. More specifically, bacteria belonging to the genus Ralstonia, the genus Aeromonas, the genus Esherichia, the genus Alcaligenes, the genus Pseudomonas, etc. may be used. Preferred is one belonging to the genus Ralstonia, and more preferred is Ralstonia eutropha.

The polyester synthase expression plasmid may be transformed into a microorganism by conventional methods. For example, an electroporation method (Current Protocols in Morecular Biology, vol. 1, pages 1.8.4, 1994), a calcium method (Lederberg. E. M. et al., J. Bacteriol. 119. 1072 (1974)), etc. may be used.

As the transformant to be preferably used in the present invention, there may be mentioned, for example, a transformant obtained by introducing each polyester synthase expression plasmid into the host Ralstonia eutropha, and the like transformants. Specifically, there may be mentioned the transformants described below, etc.
Ralstonia eutropha PHB-4/pJRDdcmBEE32d13 which is a transformant into which pJRDdcmBEE32d13 is introduced (accession number FERM P-19352, deposit date May 16, 2003),
Ralstonia eutropha PHB-4/pJRDdcm25YFEE32d13 which is a transformant into which pJRDdcm25YFEE32d13 is introduced (accession number FERM P-19353, deposit date May 16, 2003),
Ralstonia eutropha PHB-4/pJRDdcm163EE32d13 which is a transformant into which pJRDdcm163EE32d13 is introduced (accession number FERM P-19354, deposit date May 16, 2003),
Ralstonia eutropha PHB-4/pJRDdTcsEE32d13 which is a transformant into which pJRDdTcsEE32d13 is introduced (accession number FERM P-19355, deposit date May 16, 2003),
Ralstonia eutropha PHB-4/pJRDdTcEE32d13 which is a transformant into which pJRDdTcEE32d13 is introduced (accession number FERM BP-08624, the Budapest Treaty depositry transferred from the domestic depositry as of May 16, 2003),
Ralstonia eutropha PHB-4/pJRDdncEE32d13 which is a transformant into which pJRDdncEE32d13 is introduced (accession number FERM BP-08625, the Budapest Treaty depositry transferred from the domestic depositry as of May 16, 2003),
Ralstonia eutropha PHB-4/pJRDdsEE32d13 which is a transformant into which pJRDdsEE32d13 is introduced (accession number FERM P-19358, deposit date May 16, 2003),
Ralstonia eutropha PHB-4/pJRDdmsEE32d13 which is a transformant into which pJRDdmsEE32d13 is introduced (accession number FERM BP-08626, the Budapest Treaty depositry transferred from the domestic depositry as of May 16, 2003),
Ralstonia eutropha PHB-4/pJRDdmEE32d13 which is a transformant into which pJRDdmEE32d13 is introduced (accession number FERM P-19360, deposit date May 16, 2003), and
Ralstonia eutropha PHB-4/pJRDdTc171DG which is a transformant into which pJRDdTc171DG is introduced (accession number FERM BP-08623, the Budapest Treaty depositry as of February 13, 2004).

These transformants are deposited to National Institute of Advanced Industrial Science and Technology, International Patent Organism Depositary, Central 6, 1-1-1, Higashi, Tsukuba, Ibaraki, Japan.

### Production of a polyester

In the following, production of a polyester is explained.

The method of the present invention for producing a polyester is a method for producing a copolyester P(3HB-co-3HH) composed of 3-hydroxybutyric acid and 3-hydroxyhexanoic acid represented by the following formula (1) by using the above expression plasmid or the above transformant. 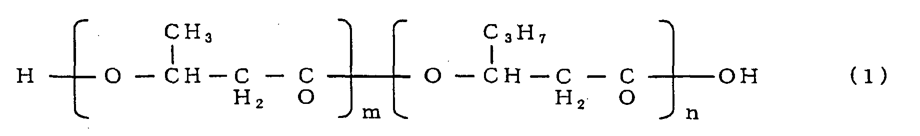 In the formula, m and n each represents an integer of 1 or more.

In producing a polyester, the above transformant may be cultured by adding a sugar, an oil or fat, or a fatty acid as a carbon source, and using a medium containing nutrient sources other than the carbon source, such as a nitrogen source, inorganic salts, and other organic nutrient sources.

For example, as a medium for culturing the transformant obtained by using bacteria belonging to the genus such as Ralstonia, Aeromonas, Esherichia, Alcaligenes or Pseudomonas as a host, a medium in which a carbon source capable of being utilized by the bacteria is added, and, in some cases, any of a nitrogen source, inorganic salts and an organic nutrient source is limited (for example, a nitrogen source is limited to 0.01 to 0.1%), or the like medium may be used.

As the sugar, there may be mentioned, for example, carbohydrates such as glucose and fructose. As the oil or fat, there may be mentioned oils or fats containing much of saturated/unsaturated fatty acids having ten or more carbon atoms, such as a coconut oil, a palm oil, a palm kernel oil, etc. As the fatty acid, there may be mentioned saturated/unsaturated fatty acids such as hexanoic acid, octanoic acid, decanoic acid, lauric acid, oleic acid, palmitic acid, linoleic acid, linolenic acid and myristic acid, or esters and salts of these fatty acids or the like fatty acid derivatives.

As the nitrogen source, there may be mentioned, for example, ammonium salts such as ammonia, ammonium chloride, ammonium sulfate and ammonium phosphate, and peptone, a meat extract, a yeast extract, etc.

As the inorganic salts, there may be mentioned, for example, potassium phosphate, potassium diphosphate, magnesium phosphate, magnesium sulfate, sodium chloride, etc.

As the other organic nutrient sources, there may be mentioned, for example, amino acids such as glycine, alanine, serine, threonine and proline; vitamins such as vitamin B1, vitamin B12 and vitamin C; etc.

Moreover, antibiotics (kanamycin, etc.) corresponding to a resistance gene existing in the vector may be added to a culture medium.

A culture temperature may be at any temperatures as long as the cell may grow up, but preferably 20 to 40°C. A culture period is not particularly restricted, but may be about 1 to 7 days.

Then, a polyester may be recovered from said cultured cell which is obtained.

In the present invention, a polyester may be recovered from cells by the following methods, for example. After completion of culture, cells are separated by a centrifuge, etc. from a culture medium, the cells are washed with distilled water, methanol, etc., and then dried. From these dried cells, a polyester is extracted using an organic solvent such as chloroform. A cell component is removed from the organic solvent solution containing the polyester by filtration, etc., and then a poor solvent such as methanol or hexane is added to the filtrate to precipitate the polyester. Moreover, a supernatant is removed by filtration, centrifugation, etc. to dry and recover the polyester.

Average molecular weight and 3HH composition (mol%) of the obtained polyester may be analyzed by a gas chromatograph method, a nuclear magnetic resonance method, etc., for example. Alternatively, as a simple method for confirming polyester production, a staining method using Nile red may be applied. That is, polyester production may be confirmed by a method comprising adding Nile red to a medium in which recombinant bacteria grow, culturing the recombinant bacteria for 1 to 7 days, and observing whether the recombinant bacteria turn red or not.

### Evaluation of an ability of conjugal mobilization

Next, evaluation of an ability of conjugal mobilization is explained. Methods for evaluating an ability of conjugal mobilization of the constructed vector include, but are not limited to, the following methods.

Firstly, a bacterium harboring a tra gene is transformed with a vector to be evaluated, and used as a donor bacterium. As the bacterium harboring a tra gene, for example, Esherichia coli S17-1 strain, etc. may be used. A recipient bacterium may be any bacteria as long as it is cultured with the donor bacterium and a replication of the vector to be evaluated may occur in the bacterium, but preferred are those having a different resistance to antibiotics from that of the donor bacterium in order to be separated from the donor bacterium after a mixed culture.

Then, a mixed culture is carried out by culturing the donor bacterium and the recipient bacterium separately in an appropriate medium beforehand to grow them, mixing them, and inoculating an appropriate medium with them.

A culture medium to be used at this time may be either a solid or liquid, and an antibiotic is not added. The culture temperature in a mixed culture may be any temperatures as long as the donor bacterium and the recipient bacterium may grow, but preferably 20 to 40°C. The culture period is not particularly restricted, but preferably 5 to 20 hours. Evaluation of an ability of conjugal mobilization includes, but is not limited to, a method comprising calculating a conjugal mobilization frequency per recipient bacterium. Methods for calculating the conjugal mobilization frequency is described in detail in Examples below.

### BRIEF DESCRIPTION OF THE DRAWINGS

Fig. 1 is a construction view of a mob region-deleted vector and a streptomycin resistance gene-deleted vector according to the present invention.
Fig. 2 is a construction view of a cos region- and oriT region-deleted vector according to the present invention.
Fig. 3 is a construction view of a mob gene region-partially deleted and cos region-deleted vector according to the present invention.
Fig. 4 is a construction view of a mob gene region-partially deleted and cos region-deleted vector according to the present invention.
Fig. 5 is a construction view of a mob A gene-mutated and cos region-deleted vector according to the present invention.
Fig. 6 is a construction view of a mob region- and oriT region-deleted vector according to the present invention.

### BEST MODE FOR CARRYING OUT THE INVENTION

Hereinafter, the present invention is described in detail by way of Examples. However, these Examples are not limitative of a technical scope of the present invention.

### (Example 1) Partial deletions of mobA and mobB genes

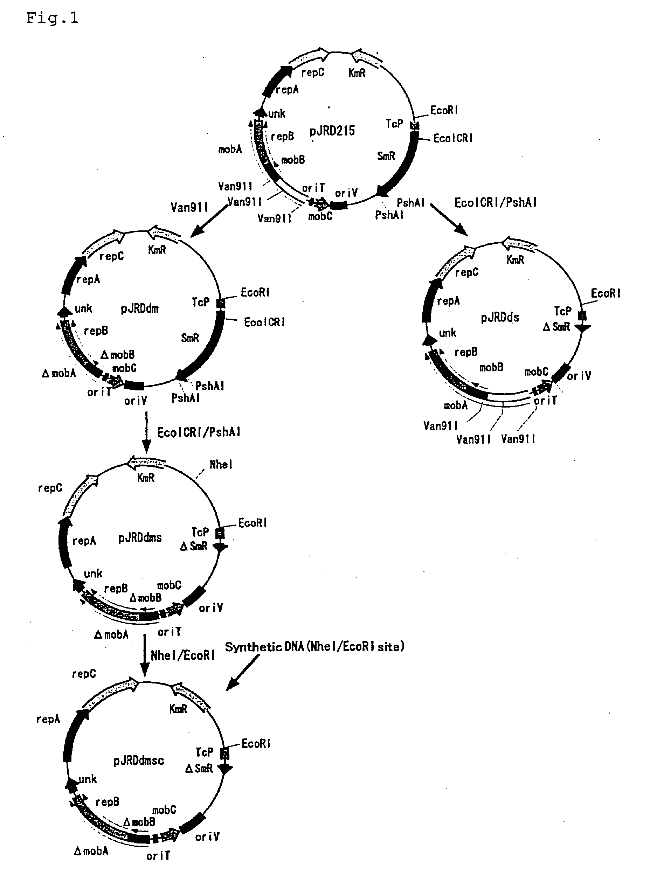

A DNA fragment of about 9.5 kb in which parts of mobA and B genes had been deleted was obtained by cutting the plasmid pJRD215 with a restriction enzyme Van91I. This fragment was subjected to self-ligation using DNA Ligation Kit Ver.1 (product of TAKARA SHUZO CO., LTD.), and a plasmid pJRDdm was obtained (Fig. 1). This plasmid pJRDdm has a deletion of the sequence from 3215 to 4075 of pJRD215.

### (Example 2) Deletions of a cos region and an oriT region

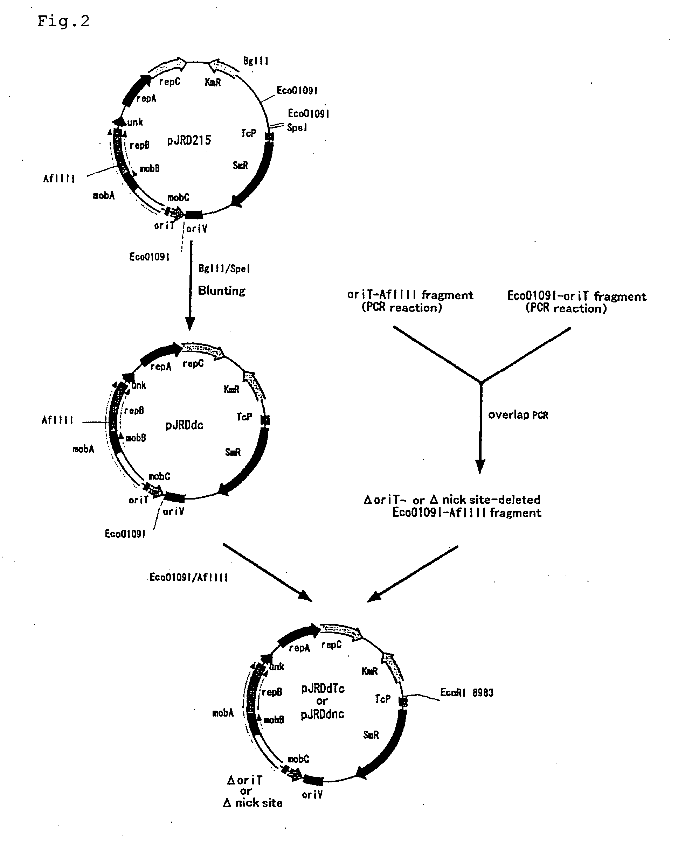

The plasmid pJRD215 was cut with restriction enzymes SpeI and BglII, and the resultant was blunted by using DNA Blunting Kit (product of TAKARA SHUZO CO., LTD.) and subjected to self-ligation. This plasmid is named as pJRDdc (Fig. 2). Said plasmid pJRDdc has a deletion of the sequence from 8915 to 10055 of pJRD215.

Next, PCR was carried out using primers shown under the SEQ ID NO:4 and 6 of the sequence listing, and the plasmid pJRDdc as a template to obtain a DNA fragment of about 0.5 kb. In a similar manner, a DNA fragment of about 2.4 kb was obtained by PCR using pJRDdc as a template and primers shown under the SEQ ID NO:5 and 7 of the sequence listing. An overlap PCR method was carried out by making use of an overlap portion of the obtained two fragments. As a polymerase, Pyrobest (product of TAKARA SHUZO CO., LTD.) was used. The fragment of about 2.8 kb obtained by the overlap PCR method was cut with restriction enzymes EcoO109I and AflIII, and ligated to the above pJRDdc cut with EcoO109I and AflIII in the same manner. This gave pJRDdTc (Fig. 2). This plasmid pJRDdTc has a deletion of the sequence from 3132 to 3169 of pJRD215 and also of its cos region.

Similarly, PCR was carried out by using primers shown under the SEQ ID NO:4 and 8 of the sequence listing, and using pJRDdc as a template to obtain a DNA fragment of about 0.5 kb. Also in the same manner, a DNA fragment of about 2.4 kb was obtained by PCR using primers shown under the SEQ ID NO:5 and 9 of the sequence listing and using pJRDdc as a template. An overlap PCR method was carried out by making use of an overlap portion of the obtained two fragments. As a polymerase, Pyrobest was used. The fragment of about 2.8 kb obtained by the overlap PCR method was cut with the restriction enzymes EcoO109I and Af1III, and ligated to the pJRDdc cut with EcoO109I and Af1III in the same manner. This gave pJRDdnc (Fig. 2). This plasmid pJRDdnc has a deletion of the sequence from 3132 to 3145 of pJRD215 and also of its cos region.

### (Example 3) A cos region deletion and a mob gene region deletion of pJRDdm, or a mutation introduction into pJRDdm

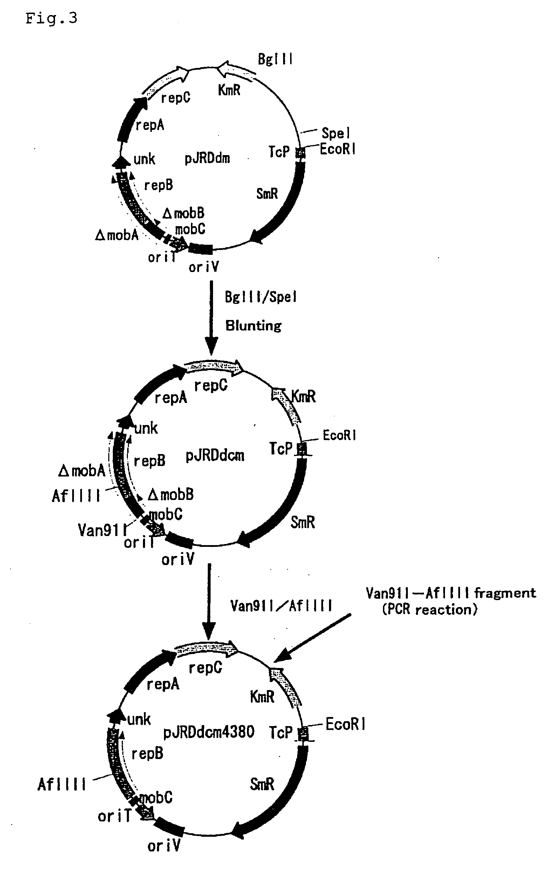

The Plasmid pJRDdm was cut with the restriction enzymes SpeI and BglII, the resultant was blunted by using DNA Blunting Kit (product of TAKARA SHUZO CO., LTD.) and subjected to self-ligation. This plasmid is named as pJRDdcm (Fig. 3).

Next, PCR was carried out using primers shown under the SEQ ID NO:5 and 10 of the sequence listing, and pJRDdcm as a template. The obtained DNA fragment was cut with the restriction enzymes Van91I and Af1III, and was ligated to pJRDdcm cut with the restriction enzymes Van91I and Af1III in the same manner, and a plasmid pJRDdcm4380 was obtained (Fig. 3).

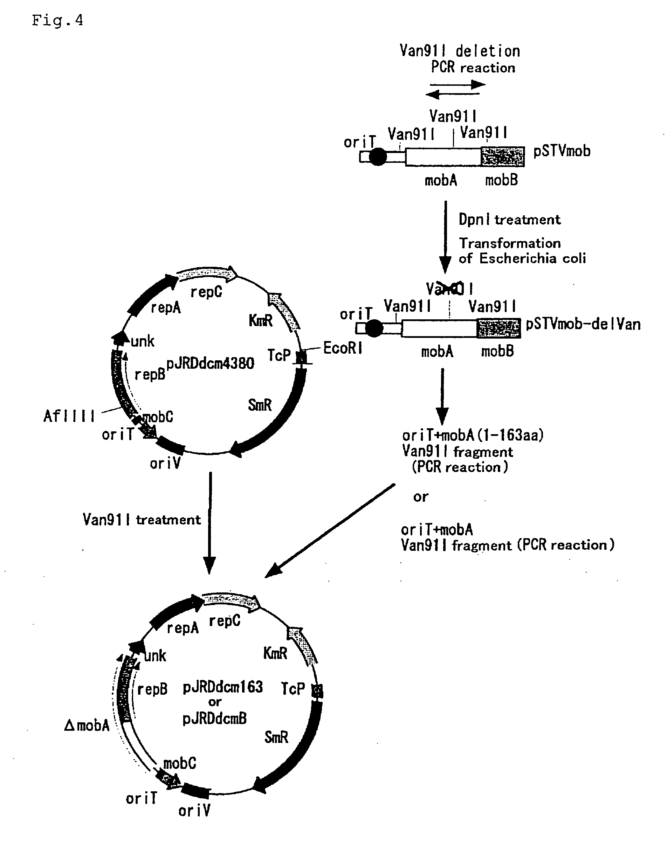

On the other hand, PCR was carried out using primers shown under the SEQ ID NO:11 and 12 of the sequence listing, and the plasmid pJRD215 as a template. As a polymerase, Pyrobest was used. The obtained DNA fragment of about 1.5 kb was cut with restriction enzymes XhoI and PstI, and inserted into pSTV28 vector (product of TAKARA SHUZO CO., LTD.) cut with the restriction enzymes XhoI and PstI to produce pSTVmob. Then, pSTVmob was amplified using PCR primers shown under the SEQ ID NO:13 and 14 of the sequence listing to delete the second Van91I site among three Van91I sites in pSTVmob. As a polymerase, Pfu (product of Stratagene Corp.) was used. After carrying out PCR, the template plasmid was cut by being added with a restriction enzyme DpnI, and Esherichia coli JM109 strain was transformed with the product. Then, a plasmid pSTVmob-delVan which is deleted one of Van91I sites was obtained from the transformant (Fig. 4).

Then, PCR was carried out using primers shown under the SEQ ID NO:15 and 16 of the sequence listing and pSTVmob-delVan as a template. As a polymerase, Pyrobest was used. The amplified fragment was cut with a restriction enzyme Van91I, and ligated to pJRDdcm4380 which had been treated with Van91I, thereby pJRDdcm163 was obtained (Fig. 4). Said plasmid pJRDdcm163 has a deletion of the sequence from 3737 to 4378 of pJRD215 and also of its cos region.

Similarly, PCR was carried out using primers shown under the SEQ ID NO:15 and 17 of the sequence listing, and pSTVmob-delVan as a template. As a polymerase, Pyrobest was used. The amplified fragment was cut with a restriction enzyme Van91I, and ligated to pJRDdcm4380 which had been treated with Van91I, thereby pJRDdcmB was obtained (Fig. 4). Said plasmid pJRDdcmB has a deletion of the sequence from 4000 to 4378 of pJRD215 and also of its cos region.

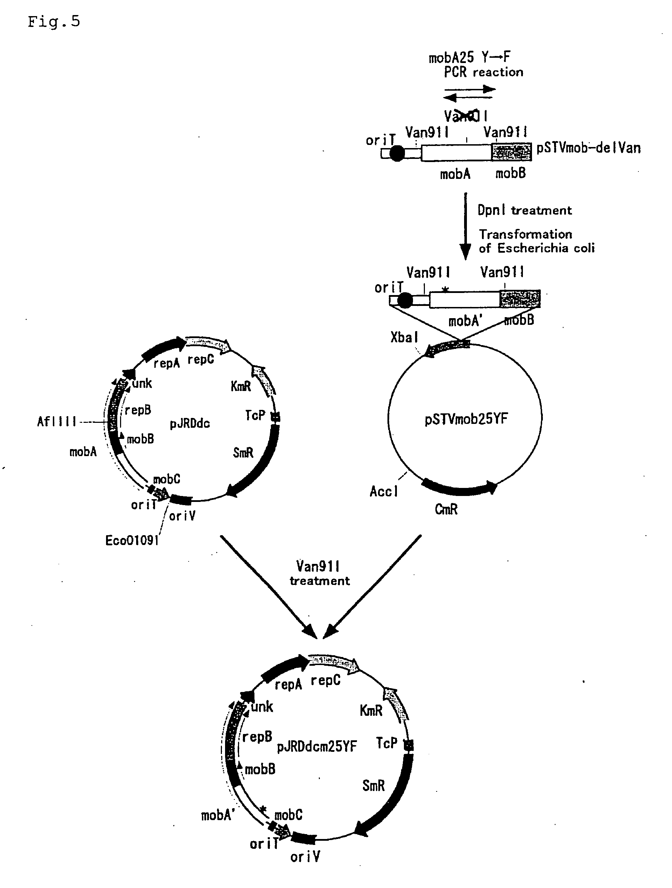

Next, the following operations were carried out to substitute thymine for 3323 adenine of pJRD215 (SEQ ID NO:1), that is, to substitute phenylalanine for the 25th amino acid residue tyrosine of a mobA gene. Amplification was carried out using pSTVmob-delVan as a template, and using PCR primers shown under the SEQ ID NO:18 and 19 of the sequence listing. As a polymerase, Pfu was used. After carrying out PCR, the template plasmid was cut by being added with a restriction enzyme DpnI, and a Esherichia coli JM109 strain was transformed with the product, thereby pSTVmob25YF was obtained from the transformant (Fig. 5). A fragment of about 0.9 kb obtained by treating the pSTVmob25YF with Van91I was ligated to pJRDdc cut with Van91I, thereby pJRDdcm25YF was obtained (Fig. 5). Said plasmid pJRDdcm25YF is one in which thymine is substituted for 3323 adenine of pJRD215, and its cos region is deleted.

### (Example 4) Deletion of a streptomycin resistance gene

The vector pJRD215 and the vectors pJRDdm, pJRDdTc, pJRDdnc, pJRDdcm163, pJRDdcmB and pJRDdcm25YF obtained in Examples 1 to 3 were cut with the restriction enzymes EcoICRI and PshAI. Thereby, about 1.5 kb of a streptomycin resistance genes (strA and strB genes) were deleted, and a blunted DNA fragment of about 8.0 kb was obtained. This fragment was subjected to self-ligation, and plasmids pJRDds, pJRDdms, pJRDdTcs, pJRDdncs, pJRDdscm163, pJRDdscmB and pJRDdscm25YF were obtained (Fig. 1). These plasmids are ones further deleted the sequence from 206 to 1690 of pJRD215 in the used plasmid.

### (Example 5) Deletions of a cos region and a multi-cloning site of pJRDdms

The plasmid pJRDdms obtained in Example 4 was cut with restriction enzymes NheI and EcoRI, and was ligated to a synthetic DNA sequence shown under the SEQ ID NO:2 (order chain) and NO:3 (reverse chain) of the sequence listing. Thereby, about 0.9 kb of the DNA containing a cos sequence and a multi-cloning site were deleted completely by substituting with a synthetic DNA 45bp, and a plasmid pJRDdmsc of about 7.1 kb was obtained (Fig. 1).

### (Example 6) Construction of a mob region- and oriT region- deleted vector

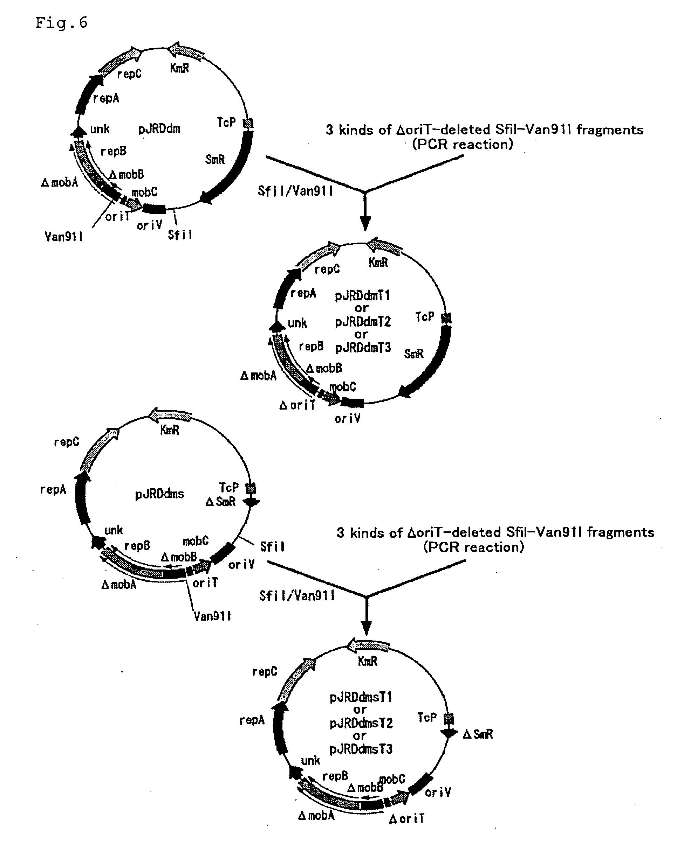

Next, PCR was carried out by using primers shown under the SEQ ID NO:20 and 21 of the sequence listing, and using pJRD215 as a template to obtain a DNA fragment of about 1 kb. As a polymerase, Pyrobest was used. This DNA fragment was cut respectively with each restriction enzymes SfiI and Van91I, and was ligated to each of the plasmids pJRDdm and pJRDdms, which had been cut with each restriction enzymes SfiI and Van91I in the same manner, thereby plasmids pJRDdmT1 and pJRDdmsT1 were obtained (Fig. 6).

Similarly, PCR was carried out using primers shown under the SEQ ID NO:20 and 22 of the sequence listing, and using pJRD215 as a template to obtain a DNA fragment of about 1 kb. This DNA fragment was cut respectively with each restriction enzymes SfiI and Van91I, and was ligated to each of the plasmids pJRDdm and pJRDdms, which had been cut with each restriction enzymes SfiI and Van91I in the same manner, thereby plasmids pJRDdmT2 and pJRDdmsT2 were obtained (Fig. 6).

Furthermore, similarly, PCR was carried out using primers shown under the SEQ ID NO:20 and 23 of the sequence listing, and pJRD215 as a template to obtain a DNA fragment of about 1 kb. This DNA fragment was cut respectively with each restriction enzymes SfiI and Van91I, and was ligated to each of the plasmids pJRDdm and pJRDdms, which had been cut with each restriction enzymes SfiI and Van91I in the same manner, thereby plasmids pJRDdmT3 and pJRDdmsT3 were obtained (Fig. 6).

### (Example 7) Construction of an expression plasmid for polyester production and construction of a transformant

Each fragment of N149S-modified gene, D171G-modified gene and F353T-modified gene was prepared by PCR. D171G modification, for example, is a modification in which glycine is substituted for 171st amino acid aspartic acid of an Aeromonas caviae-derived PHA synthase. Therefore, a gene fragment EE32d13 derived from Aeromonas caviae, disclosed in Japanese Kokai Publication Hei-10-108682, was subcloned into an EcoRI site of pUC19 in advance, and then PCR was carried out using synthetic DNAs shown under the SEQ IDs:24 and 25 as primers. PCR was carried out with 25 cycles each comprising (1) at 94°C for 2 min., (2) at 94°C for 30 sec., (3) at 55°C for 30 sec. and (4) at 72°C for 2 min., and then (5) at 72°C for 5 min. Ex Taq polymerase (product of TAKARA BIO) was used as a polymerase.

Each of the vectors pJRDds, pJRDdm, pJRDdms, pJRDdTc, pJRDdTcs, pJRDdnc, pJRDdcm163, pJRDdcmB and pJRDdcm25YF obtained in Examples 1 to 5 was cut with the restriction enzyme EcoRI. Then, the gene fragment EE32d13 derived from Aeromonas caviae, or a fragment of N149S-modified gene, D171G-modified gene or F353T-modified gene, was respectively inserted into EcoRI sites of the above-mentioned plasmids to produce expression plasmids pJRDdsEE32d13, pJRDdmEE32d13, pJRDdmsEE32d13, pJRDdTcEE32d13, pJRDdTc149NS, pJRDdTc171DG, pJRDdTc353FT, pJRDdTcsEE32d13, pJRDdncEE32d13, pJRDdcm163EE32d13, pJRDdcmBEE32d13 and pJRDdcm25YFEE32d13.

All of transformant strains of Ralstonia eutropha containing each of these expression plasmid species were produced by an electrical pulse method. That is, as gene transferring device, Gene Pulser manufactured by Bio-Rad Laboratories was used, and as a cuvette, the one having a gap of 0.2 cm also manufactured by Bio-Rad Laboratories was used. The cuvette was charged with 400 µl of competent cells and 20 µl of plasmids, placed in the pulse device, and then electrical pulse was applied in conditions of an electric capacity of 25 µF, a voltage of 1.5 kV and a resistance value of 800 Ω. After the application of pulse, an aqueous fluid containing bacteria in the cuvette was cultured with shaking in Nutrient Broth medium (product of DIFCO Laboratories) at 30°C for 3 hours, and then was cultured in a selected plate (Nutrient Agar medium (product of DIFCO Laboratories), kanamycin 100 mg/l) at 30°C for 2 days to obtain transformants.

These transformants were deposited to National Institute of Advanced Industrial Science and Technology, International Patent Organism Depositary (pJRDdcmBEE32d13 transformant = FERM P-19352, pJRDdcm25YFEE32d13 transformant = FERM P-19353, pJRDdcm163EE32d13 transformant = FERM P-19354, pJRDdTcsEE32d13 transformant = FERM P-19355, pJRDdTcEE32d13 transformant = FERM BP-08624, pJRDdncEE32d13 transformant = FERM BP-08625, pJRDdsEE32d13 transformant = FERM P-19358, pJRDdmsEE32d13 transformant = FERM BP-08626, pJRDdmEE32d13 transformant = FERM P-19360 and pJRDdTc171DG transformant = FERM BP-08623).

### (Example 8) Production of a polyester using a transformant

The strains of transformants obtained in Example 7 were seeded in a Nile red-containing medium (disodium hydrogenphosphate dodecahydrate 9 g, potassium dihydrogenphosphate 1.5 g, ammonium chloride 0.05 g, magnesium sulfate heptahydrate 0.02 g, fructose 0.5 g, cobalt chloride hexahydrate 0.25 ppm, iron(III) chloride hexahydrate 16 ppm, calcium chloride dihydrate 10.3 ppm, nickel chloride hexahydrate 0.12 ppm, copper sulfate pentahydrate 0.16 ppm, Nile red 0.5 mg, and agar 15 g/l L), and cultured at 30°C for one week. After the culture, all of the strains turned red, and a polyester was found to be accumulated in cells.

### (Example 9) Evaluation of an ability of conjugal mobilization of a transformant

Among the expression plasmids described in Example 7, an ability of conjugal mobilization was evaluated for pJRDdmEE32d13, pJRDdmsEE32d13, pJRDdTcEE32d13 and pJRDdncEE32d13. As a control, pJRDEE32d13 was used.

Esherichia coli S17-1 strains transformed with each of these expression plasmids were used as donor bacteria, and Esherichia coli XL10-gold strain was used as a recipient bacterium. Each of these donor bacteria and the recipient bacterium was seeded to a TB medium (tryptone 1.2%, yeast extract 2.4% and glycerol 0.4%), and was cultured at 37°C overnight. A sterilized nitrocellulose filter was put onto an LB plate (tryptone 1%, yeast extract 0.50, NaCl 1% and agar 1.5%), and a culture solution was mixed with 10 µl of each of the donor bacterium and 10 µl of the recipient bacterium, and the mixed culture solution was inoculated onto the nitrocellulose filter. After the solution was cultured for 5 to 15 hours at 37°C, the nitrocellulose filter was moved to a tube charged with 1 ml of saline, and suspended sufficiently to float the bacteria held on the nitrocellulose filter.

The thus obtained suspension of mixed bacteria was diluted to be 10° to 10⁷-fold amount and was plated by 100 µl on an LB plate containing 50 µg/ml of kanamycin and 20 µg/ml of chloramphenicol, and on an LB plate containing 20 µg/ml of chloramphenicol alone, respectively, followed by culturing them at 37°C overnight. After the culture, the number of the respective emerged colonies was counted. The results are shown in Table 1.

**Table 1**

| | | | | | | | | | |
|---|---|---|---|---|---|---|---|---|---|
| The number of emerged colonies in conjugal mobilization evaluation using the expression plasmids | | | | | | | | | |

| | Cell suspension dilution ratio | × 10⁰ | × 10⁻¹ | × 10⁻² | × 10⁻³ | × 10⁻⁴ | × 10⁻⁵ | × 10⁻⁶ | × 10⁻⁷ |
|---|---|---|---|---|---|---|---|---|---|
| pJRDdm EE32d13 | LB(Cm) | - | - | - | - | - | 1007 | 111 | - |
| | LB(Km,Cm) | 9 | 0 | - | - | - | - | - | - |
| pJRDdms EE32d13 | LB(Cm) | - | - | - | - | - | - | about 1800 | 183 |
| | LB(Km,Cm) | 13 | - | - | - | - | - | - | - |
| pJRDdTc EE32d13 | LB(Cm) | - | - | - | - | - | 301 | 32 | - |
| | LB(Km,Cm) | 0 | - | - | - | - | - | - | - |
| pJRDdnc EE32d13 | LB(Cm) | - | - | - | - | - | 420 | 40 | - |
| | LB(Km,Cm) | 0 | - | - | - | - | - | - | - |
| LB(Cm) represents an LB plate containing chloramphenicol, | | | | | | | | | |
| LB(Km, Cm) represents an LB plate containing kanamycine and chloramphenicol, and - represents "not completed with". | | | | | | | | | |

Esherichia coli S17-1 strain is sensitive to chloramphenicol and kanamycin. On the other hand, Esherichia coli XL10-gold strain is resistant to chloramphenicol and sensitive to kanamycin. Since all the expression plasmids used for transforming Esherichia coli S17-1 strains contain a kanamycin resistance gene, the bacteria harboring the expression plasmid are resistant to kanamycin. Therefore, on the LB plate containing 20 µg/ml of chloramphenicol alone, the total number of Esherichia coli XL10-gold strains may be detected, and on the LB plate containing 50 µg/ml of kanamycin and 20 µg/ml of chloramphenicol, Esherichia coli XL10-gold strain transformed with an expression plasmid may be detected.

Then, the conjugal mobilization frequency per recipient bacterium is calculated by dividing the number of Esherichia coli XL10-gold strains, in which an expression plasmid is introduced by conjugal mobilization, contained in 100 µl of a cell suspension, by the total number of Esherichia coli XL10-gold strains. Additionally, when the number of XL10-gold strains introduced is 0, the number of XL10-gold strains introduced is set as 1, temporarily, and conjugal mobilization frequency per recipient bacterium is calculated to represent as being less than the obtained conjugal mobilization frequency.

In the following, the actual calculated values are shown.

From the results shown in Table 1, the conjugal mobilization frequency per recipient bacterium in the case pJRDdmEE32d13 coexists with a tra gene was 9/1007 x 10⁻⁵ = 8.9 x 10⁻⁸; the conjugal mobilization frequency per recipient bacterium in the case pJRDdmsEE32d13 coexists with a tra gene was 13/183 x 10⁻⁷ = 7.1 x 10⁻⁹; the conjugal mobilization frequency per recipient bacterium in the case pJRDdTcEE32d13 coexists with a tra gene was less than 1/301 x 10⁻⁵ = 3.3 x 10⁻⁸; and the conjugal mobilization frequency per recipient bacterium in the case pJRDdncEE32d13 coexists with a tra gene was less than 1/420 x 10⁻⁵ = 2.4 x 10⁻⁸.

The conjugal mobilization frequency per recipient bacterium of pJRD215EE32d13 tested separately in the same manner was 318/513 x 10⁻³ = 6.2 x 10⁻⁴. Therefore, the conjugal mobilization frequency of pJRDdmEE32d13, pJRDdmsEE32d13, pJRDdTcEE32d13 and pJRDdncEE32d13 dropped to the level of not more than 1/10⁴ to 1/10⁵, it is considered that there is substantially no ability of conjugal mobilization even when they coexist with a tra gene.

### INDUSTRIAL APPLICABILITY

As mentioned above, it becomes possible to produce a polyester more safely in an industrial production and/or efficiently in a low cost by a genetic engineering technique using a novel vector, especially one capable of being replicated in Ralstonia eutropha.

## Claims

1. A polyester synthase expression plasmid
which has partly or totally lost ability of conjugal mobilization.

2. The polyester synthase expression plasmid according to Claim 1,
wherein a vector is pJRD215 (SEQ ID NO:1).

3. The polyester synthase expression plasmid according to Claim 1 or 2,
wherein a polyester synthase gene contained in the plasmid is one or more of genes among the following (1) to (5):
(1) a polyester synthase gene derived from Aeromonas caviae;
(2) an Aeromonas caviae-derived polyester synthase gene,
wherein serine is substituted for 149th amino acid asparagine;
(3) an Aeromonas caviae-derived polyester synthase gene,
wherein glycine is substituted for 171st amino acid aspartic acid;
(4) an Aeromonas caviae-derived polyester synthase gene,
wherein threonine is substituted for 353rd amino acid phenylalanine; and
(5) an Aeromonas caviae-derived polyester synthase gene comprising two or more of the above amino acid substitutions of (2) to (4).

4. The polyester synthase expression plasmid according to any one of Claims 1 to 3,
wherein the partly or totally lost ability of conjugal mobilization is caused by a deletion or mutation of a mob gene region.

5. The polyester synthase expression plasmid according to Claim 4,
wherein the deletion of a mob gene region is a deletion of the sequence from 3215 to 4075 of pJRD215 (SEQ ID NO:1).

6. The polyester synthase expression plasmid according to Claim 4,
wherein the deletion of a mob gene region is a deletion of the sequence from 3737 to 4378 of pJRD215 (SEQ ID NO:1).

7. The polyester synthase expression plasmid according to Claim 4,
wherein the deletion of a mob gene region is a deletion of the sequence from 4000 to 4378 of pJRD215 (SEQ ID NO:1).

8. The polyester synthase expression plasmid according to Claim 4,
wherein the mutation of a mob gene region is a substitution of thymine for 3323 adenine of pJRD215 (SEQ ID NO:1).

9. The polyester synthase expression plasmid according to any one of Claims 1 to 3,
wherein the partly or totally lost ability of conjugal mobilization is caused by a deletion of an oriT region.

10. The polyester synthase expression plasmid according to Claim 9,
wherein the deletion of an oriT region is a deletion of the sequence from 3132 to 3145 of pJRD215 (SEQ ID NO:1).

11. The polyester synthase expression plasmid according to Claim 9,
wherein the deletion of an oriT region is a deletion of the sequence from 3132 to 3169 of pJRD215 (SEQ ID NO:1).

12. The polyester synthase expression plasmid according to any one of Claims 1 to 3,
wherein the partly or totally lost ability of conjugal mobilization is caused by deletions of a mob gene region and an oriT region.

13. The polyester synthase expression plasmid according to Claim 12,
wherein the deletion of a mob gene region is a deletion of the sequence from 3215 to 4075 of pJRD215 (SEQ ID NO:1), and the deletion of an oriT region is a deletion of the sequence from 3132 to 3145 of pJRD215 (SEQ ID NO:1).

14. The polyester synthase expression plasmid according to Claim 12,
wherein the deletion of a mob gene region is a deletion of the sequence from 3215 to 4075 of pJRD215 (SEQ ID NO:1), and the deletion of an oriT region is a deletion of the sequence from 3132 to 3169 of pJRD215 (SEQ IB NO:1).

15. The polyester synthase expression plasmid according to Claim 12,
wherein the deletion of a mob gene region is a deletion of the sequence from 3215 to 4075 of pJRD215 (SEQ ID NO:1), and the deletion of an oriT region is a deletion of the sequence from 3132 to 3178 of pJRD215 (SEQ ID NO:1).

16. The polyester synthase expression plasmid according to Claim 12,
wherein the deletion of a mob gene region is a deletion of the sequence from 3215 to 4075 of pJRD215 (SEQ ID NO:1), and the deletion of an oriT region is a deletion of the sequence from 3132 to 3214 of pJRD215 (SEQ ID NO:1).

17. The polyester synthase expression plasmid according to Claim 12,
wherein the deletion of a mob gene region is a deletion of the sequence from 3215 to 4075 of pJRD215 (SEQ ID NO:1), and the deletion of an oriT region is a deletion of the sequence from 3095 to 3214 of pJRD215 (SEQ ID NO:1).

18. A polyester synthase expression plasmid
which comprises a deletion of a streptomycin resistance gene region.

19. The polyester synthase expression plasmid according to Claim 18,
wherein a vector is pJRD215 (SEQ ID NO:1).

20. The polyester synthase expression plasmid according to Claims 18 or 19,
wherein a polyester synthase gene contained in the plasmid is one or more of genes among the following (1) to (5) :
(1) a polyester synthase gene derived from Aeromonas caviae;
(2) an Aeromonas caviae-derived polyester synthase gene, wherein serine is substituted for 149th amino acid asparagine;
(3) an Aeromonas caviae-derived polyester synthase gene,
wherein glycine is substituted for 171st amino acid aspartic acid;
(4) an Aeromonas caviae-derived polyester synthase gene,
wherein threonine is substituted for 353rd amino acid phenylalanine; and
(5) an Aeromonas caviae-derived polyester synthase gene comprising two or more of the above amino acid substitutions of (2) to (4).

21. The polyester synthase expression plasmid according to any one of Claims 18 to 20,
wherein the deletion of a streptomycin resistance gene region is a deletion of the sequence from 206 to 1690 of pJRD215 (SEQ ID NO:1).

22. A polyester synthase expression plasmid
which has partly or totally lost ability of conjugal mobilization, and comprises a deletion of a streptomycin resistance gene.

23. The polyester synthase expression plasmid according to Claim 22,
wherein a vector is pJRD215 (SEQ ID NO:1).

24. The polyester synthase expression plasmid according to Claim 22 or 23,
wherein a polyester synthase gene contained in the plasmid is one or more of genes among the following (1) to (5) :
(1) a polyester synthase gene derived from Aeromonas caviae;
(2) an Aeromonas caviae-derived polyester synthase gene,
wherein serine is substituted for 149th amino acid asparagine;
(3) an Aeromonas caviae-derived polyester synthase gene,
wherein glycine is substituted for 171st amino acid aspartic acid;
(4) an Aeromonas caviae-derived polyester synthase gene,
wherein threonine is substituted for 353rd amino acid phenylalanine; and
(5) an Aeromonas caviae-derived polyester synthase gene comprising two or more of the above amino acid substitutions of (2) to (4).

25. The polyester synthase expression plasmid according to any one of Claims 22 to 24,
wherein the partly or totally lost ability of conjugal mobilization is caused by a deletion or mutation of a mob gene region.

26. The polyester synthase expression plasmid according to Claim 25,
wherein the deletion of a mob gene region is a deletion of the sequence from 3215 to 4075 of pJRD215 (SEQ ID NO:1).

27. The polyester synthase expression plasmid according to Claim 25,
wherein the deletion of a mob gene region is a deletion of the sequence from 3737 to 4378 of pJRD215 (SEQ ID NO:1).

28. The polyester synthase expression plasmid according to Claim 25,
wherein the deletion of a mob gene region is a deletion of the sequence from 4000 to 4378 of pJRD215 (SEQ ID NO:1).

29. The polyester synthase expression plasmid according to Claim 25,
wherein the mutation of a mob gene region is a substitution of thymine for 3323 adenine of pJRD215 (SEQ ID NO:1).

30. The polyester synthase expression plasmid according to any one of Claims 22 to 24,
wherein the partly or totally lost ability of conjugal mobilization is caused by a deletion of an oriT region.

31. The polyester synthase expression plasmid according to Claim 30,
wherein the deletion of an oriT region is a deletion of the sequence from 3132 to 3145 of pJRD215 (SEQ ID NO:1).

32. The polyester synthase expression plasmid according to Claim 30,
wherein the deletion of an oriT region is a deletion of the sequence from 3132 to 3169 of pJRD215 (SEQ ID NO:1).

33. The polyester synthase expression plasmid according to any one of Claims 22 to 24,
wherein the partly or totally lost ability of conjugal mobilization is caused by deletions of a mob gene region and an oriT region.

34. The polyester synthase expression plasmid according to Claim 33,
wherein the deletion of a mob gene region is a deletion of the sequence from 3215 to 4075 of pJRD215 (SEQ ID NO:1), and the deletion of an oriT region is a deletion of the sequence from 3132 to 3145 of pJRD215 (SEQ ID NO:1).

35. The polyester synthase expression plasmid according to Claim 33,
wherein the deletion of a mob gene region is a deletion of the sequence from 3215 to 4075 of pJRD215 (SEQ ID NO:1), and the deletion of an oriT region is a deletion of the sequence from 3132 to 3169 of pJRD215 (SEQ ID NO:1).

36. The polyester synthase expression plasmid according to Claim 33,
wherein the deletion of a mob gene region is a deletion of the sequence from 3215 to 4075 of pJRD215 (SEQ ID NO:1), and the deletion of an oriT region is a deletion of the sequence from 3132 to 3178 of pJRD215 (SEQ ID NO:1).

37. The polyester synthase expression plasmid according to Claim 33,
wherein the deletion of a mob gene region is a deletion of the sequence from 3215 to 4075 of pJRD215 (SEQ ID NO:1), and the deletion of an oriT region is a deletion of the sequence from 3132 to 3214 of pJRD215 (SEQ ID NO:1).

38. The polyester synthase expression plasmid according to Claim 33,
wherein the deletion of a mob gene region is a deletion of the sequence from 3215 to 4075 of pJRD215 (SEQ ID NO:1), and the deletion of an oriT region is a deletion of the sequence from 3095 to 3214 of pJRD215 (SEQ ID NO:1).

39. The polyester synthase expression plasmid according to any one of Claims 22 to 24,
wherein the deletion of a streptomycin resistance gene region is a deletion of the sequence from 206 to 1690 of pJRD215 (SEQ ID NO:1).

40. The polyester synthase expression plasmid according to any one of Claims 1 to 39,
which further comprises a deleted cos region.

41. The polyester synthase expression plasmid according to Claim 40,
wherein the deleted cos region is the sequence from 9237 to 10127 of pJRD215 (SEQ ID NO:1).

42. The polyester synthase expression plasmid according to Claim 40,
wherein the deleted cos region is the sequence from 8915 to 10055 of pJRD215 (SEQ ID NO:1).

43. A transformant
which is transformed with the polyester synthase expression plasmid according to any one of Claims 1 to 42.

44. The transformant according to Claim 43,
wherein a host is Ralstonia eutropha.

45. A method for producing a polyester
which comprises using the expression plasmid or the transformant according to any one of Claims 1 to 44,
said polyester being a copolyester P(3 HB-co-3HH) composed of 3-hydroxybutyric acid and 3-hydroxyhexanoic acid represented by the following formula (1); 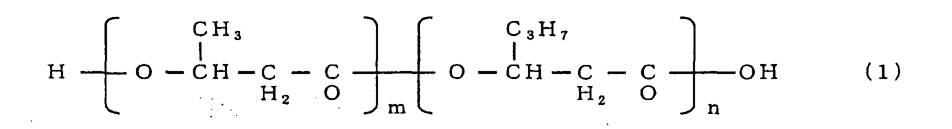 in the formula, m and n each represents an integer of 1 or more.
